# EUROPÄISCHE PATENTANMELDUNG

(11) **EP 4 015 020 A2**
(43) Veröffentlichungstag der Anmeldung: **22.06.2022**
(21) Anmeldenummer: 21204409.3
(22) Anmeldetag: 25.10.2021
(51) Int. Cl.: A61M 5/20, A61D 7/00, A61M 5/30, A61M 5/315, A61M 39/24

(54) **VORRICHTUNG ZUM APPLIZIEREN EINES FLUIDS**

(30) Priorität: 18.12.2020 DE 102020134181
(71) Anmelder: Henke-Sass, Wolf GmbH, 78532 Tuttlingen (DE)
(72) Erfinder: ALTERMANN, Frank, 78532 Tuttlingen (DE); SEEH, Daniel, 78194 Immendingen (DE)
(74) Vertreter: Patentanwälte Geyer, Fehners & Partner mbB

(57) **Zusammenfassung**

Es wird eine Vorrichtung zum Applizieren eines Fluids bereitgestellt, mit
einem Zylinder (13), der ein offenes Abgabeende (14) aufweist,
einem im Zylinder (13) zwischen einer vorderen und hinteren Endposition verschiebbaren Kolben (25), der mit einer Kolbenstange (27) verbunden ist, die entlang einer ersten Richtung über ein dem offenen Abgabeende (14) entgegengesetztes hinteres Ende (60)
des Zylinders (13) heraussteht,
einem das offene Abgabeende (14) verschließenden ersten Ventil (15),
einem mit dem Zylinder (13) verbundenen Zuführkanal (21),
und einem den Zuführkanal (21) verschließenden zweiten Ventil (20),
wobei bei geöffnetem Ventil (20) zu applizierendes Fluid über den Zuführkanal (21) in den Zylinder (13) gebracht werden kann, und
wobei eine Bewegung des Kolbens (26) entgegen der ersten Richtung zum offenen Abgabeende (14) hin einen Überdruck im Zylinder (13) erzeugt, so dass bei geöffnetem ersten Ventil (15) das zu applizierende Fluid im Zylinder (13) über das offene Abgabeende (14) zum Applizieren abgegeben wird,
wobei zur Abdichtung des hinteren Endes (60) des Zylinders (13) zwischen dem Kolben (26) und dem Zylinder (13) ein erstes Dichtelement (61) und ein davon entlang der ersten Richtung beabstandetes zweites Dichtelement (62) angeordnet sind,
wobei das erste Dichtelement (61) als einfachwirkendes Dichtelement ausgebildet ist,
das das hintere Ende (60) des Zylinders (13) gegenüber Überdruck im Zylinder (13) abdichtet, und
wobei das zweite Dichtelement (62) als einfachwirkendes oder doppelwirkendes Dichtelement ausgebildet ist, das das hintere Ende (60) des Zylinders (13) mindestens gegenüber Unterdruck im Zylinder (13) abdichtet.

## Beschreibung

Die vorliegende Erfindung betrifft eine Vorrichtung zum Applizieren eines Fluids, die beispielsweise als nadellose Selbstfüllerspritze ausgebildet sein kann, mit der Tieren ein flüssiges Medikament, ein flüssiges Arzneimittel, ein flüssiger Impfstoff oder dergleichen (insbesondere intramuskulär) verabreicht werden kann.

Eine solche Vorrichtung zum Applizieren eines Fluids soll einerseits möglichst leicht und somit für einen Benutzer lange mit einer Hand tragbar sein und gleichzeitig das gewünschte (insbesondere nadellose intramuskuläre) Injizieren ermöglichen.

Aufgabe der Erfindung ist es daher, eine solche Vorrichtung zum Applizieren eines Fluids bereitzustellen.

Die Erfindung ist im Anspruch 1 definiert. Vorteilhafte Ausgestaltungen sind in den abhängigen Ansprüchen angegeben.

Da die erfindungsgemäße Vorrichtung zum Applizieren eines Fluids das erste Dichtelement, das als einfachwirkendes Dichtelement ausgebildet ist, und das davon beabstandete zweite Dichtelement, das als einfachwirkendes oder doppelwirkendes Dichtelement ausgebildet ist, aufweist, ist eine gute Abdichtung des hinteren Endes des Zylinders sowohl für hohe, stark schwellende Drücke sowie für Unterdruck möglich. So bewirkt das erste Dichtelement ein Abdichten des hinteren Endes des Zylinders gegenüber Überdruck, der beim Applizieren des Fluids auftritt. Ferner führt das Vorsehen des zweiten Dichtelementes dazu, dass das hintere Ende des Zylinders gegenüber Unterdruck im Zylinder abgedichtet ist, der beim Ansaugen von neuem Fluid für den nächsten Applizierungsvorgang auftritt.

Somit kann man sagen, dass eine Tandem-Dichtung vorliegt, die zur Abdichtung des hinteren Endes des Zylinders gegen Überdruck (mittels des ersten Dichtelementes) und zur Abdichtung des hinteren Endes des Zylinders gegen Unterdruck (mittels des zweiten Dichtelementes) optimiert ist.

Unter einem einfachwirkenden ersten Dichtelement wird hier insbesondere ein solches Dichtelement verstanden, das das hintere Ende des Zylinders gegen Überdruck im Zylinder abdichtet und das das hintere Ende des Zylinders nicht gegen Unterdruck im Zylinder (und somit Überdruck außerhalb am hinteren Ende des Zylinders) abdichtet. Es wird somit nur der Überdruck im Zylinder gegenüber dem hinteren Ende abgedichtet.

Unter einem einfachwirkenden zweiten Dichtelement wird insbesondere ein solches Dichtelement verstanden, das das hintere Ende des Zylinders gegen Unterdruck im Zylinder (und somit gegen Überdruck außerhalb des Zylinders am hinteren Ende) abdichtet. Ein doppelwirkendes zweites Dichtelement ist hier insbesondere ein solches Dichtelement, das das hintere Ende des Zylinders gegenüber Überdruck und Unterdruck im Zylinder abdichtet.

Das erste Dichtelement kann insbesondere einen U-förmigen oder V-förmigen Querschnitt aufweisen, wobei die Öffnung des U-förmigen oder V-förmigen Querschnitts in Richtung zum offenen Abgabeende hin weist.

Das erste Dichtelement kann ferner ein Spannmittel (wie z.B. eine Feder) aufweisen, die die beiden Schenkel des U-förmigen oder V-förmigen Querschnitts in radialer Richtung auseinander drückt.

Für das erste Dichtelement kann als Material Kunststoff, ein Polymer, ein Fluorpolymer, ein Polyurethan (z.B. ein Spritz- oder Gieß-Polyurethan), ein Elastomer oder PTFE (Polytetrafluorethylen) verwendet werden. Diese Materialien können Füllstoffe (z.B. Kohlefasern oder Glasfasern) und/oder Zusatzstoffe enthalten, um die gewünschten Eigenschaften des Dichtelementes einzustellen. So kann das erste Dichtelement aus PTFE oder einem PTFE Compound hergestellt sein. Unter einem PTFE Compound wird insbesondere eine Mischung von PTFE mit mindestens einem weiteren Stoff (wie z.B. Kohle, Kohlefasern, Ruß, Glasfasern, organische Füllstoffe, Metalle, Metallliegierungen etc.) verstanden. Falls eine Feder vorgesehen ist, ist diese bevorzugt aus Metall.

Das erste Dichtelement kann einen ringförmigen Dichtteil sowie in Federelement aufweisen, das den ringförmigen Dichtteil mit einer radial nach innen gerichteten Spannung beaufschlagt. Damit kann der ringförmige Dichtteil im eingebauten Zustand mit einer radial nach innen gerichteten Vorspannung beaufschlagt sein.

Der ringförmige Dichtteil kann aus einem Thermoplast und das Federelement kann aus einem Elastomer hergestellt sein. Das Federelement kann insbesondere als O-Ring ausgebildet sein.

Das zweite Dichtelement kann in gleicher Weise wie das erste Dichtelement ausgebildet sein. Es ist dann relativ zum ersten Dichtelement um 180° gedreht angeordnet (die Öffnung des U-förmigen oder V-förmigen Querschnitts weist weg vom offenen Abgabeende und weist somit hin zum hinteren Ende des Zylinders), um die gewünschte Abdichtung des hinteren Endes des Zylinders gegenüber Unterdruck im Zylinder zu gewährleisten.

Ferner ist es möglich, dass das zweite Dichtelement als doppelwirkendes Dichtelement ausgebildet ist. In diesem Fall kann es beispielsweise als O-Ring ausgebildet sein.

Für das zweite Dichtelement können die gleichen Materialien wie für das erste Dichtelement verwendet werden. Bevorzugt können z.B. Elastomere (z.B. Fluorelastomer), PTFE oder PTFE-Compounds verwendet werden.

Das erste und zweite Dichtelement können am Zylinder angeordnet bzw. vorgesehen sein, so dass sich der Kolben relativ zu den Dichtelementen bewegt. Bei einer solchen Anordnung werden die Dichtelemente häufig als Stangendichtungen bezeichnet.

Alternativ ist es möglich, dass das erste und zweite Dichtelement am Kolben befestigt sind, so dass sich der Kolben zusammen mit den Dichtelementen im Zylinder bewegt. Bei einer solchen Anordnung werden die Dichtelemente häufig als Kolbendichtungen bezeichnet.

Es ist ferner möglich, einen Schmierring vorzusehen, der entlang der ersten Richtung relativ zum zweiten Dichtelement beabstandet ist. Der Schmierring kann beispielsweise als Filzring ausgebildet oder aus einem schwammförmigen Material gebildet sein. Ferner kann der Schmierring mit Öl oder Fett beaufschlagt sein. Damit wird eine gute Schmierung des Kolbens bei der Bewegung im Zylinder gewährleistet.

Der Zylinder kann einteilig, mehrteilig und insbesondere zweiteilig ausgebildet sein. Dabei kann der Zylinder einen hinteren Zylinderteil umfassen, dessen hinteres Ende das hintere Ende des Zylinders bildet. In dem hinteren Zylinderteil können das erste und zweite Dichtelement angeordnet sein. Ferner kann, sofern ein Schmierring vorgesehen ist, dieser ebenfalls im hinteren Zylinderteil angeordnet sein.

Das hintere Zylinderteil kann mit einem weiteren Teil des Zylinders (beispielsweise dem vorderen Zylinderteil) verbunden sein. Die Verbindung kann z.B. eine lösbare Verbindung sein. Sie kann z.B. insbesondere als Schnappverschluss oder Schraubverbindung ausgebildet sein.

Die Bewegung des Kolbens im Zylinder kann manuell oder motorbetrieben erfolgen. Es kann ein Motor vorgesehen sein, der sowohl die Bewegung des Kolbens weg vom offenen Abgabeende als auch die Bewegung hin zum offenen Abgabeende durchführt. Alternativ kann der Motor nur die Bewegung des Kolbens weg vom offenen Abgabeende bewirken, wobei dabei eine Spanneinheit (z.B. eine oder mehrere Federn) einer Spannvorrichtung gespannt wird. Zum Applizieren wird die Spanneinheit freigegeben, so dass die Spannenergie in eine Bewegung des Kolbens zum offenen Abgabeende hin umgesetzt wird. Damit kann ein hoher Druck aufgebaut werden, der insbesondere bei einem nadellosen Applizieren des Fluids gewünscht ist.

Die Spannvorrichtung kann eine mittels eines Motors drehbare Rampe mit einer sich entlang einer Schraubenlinie erstreckenden Rampenbahn aufweisen, wobei die Rampenbahn von einem ersten Plateau entlang eines Steigungsbereiches zu einem zweiten Plateau ansteigt und vom zweiten Plateau über eine Sprungflanke zum ersten Plateau abfällt, wobei die Rampenbahn einen das zweite Plateau und die Sprungflanke verbindenden Übergangsbereich aufweist. Die Spannvorrichtung kann ferner eine die Rampenbahn kontaktierende Walze, die in einem Mitnehmer, der mit der Kolbenstange verbunden ist, drehbar gelagert ist, aufweisen, so dass bei Drehung der Rampe entlang einer ersten Drehrichtung die Rampenbahn unter der sich dadurch drehenden Walze durchläuft. Für den Spannvorgang kann die Rampenbahn entlang der ersten Drehrichtung so gedreht werden, dass die Walze auf dem Steigungsbereich bis zum zweiten Plateau läuft und dadurch der Kolben in seine hintere Endposition bewegt wird, wobei die Spanneinheit gespannt wird. Für den Abgabevorgang kann die Spannvorrichtung die Rampenbahn ausgehend von einem Kontakt der Walze mit dem zweiten Plateau entlang der ersten Drehrichtung drehen, bis die Walze über den Übergangsbereich läuft und aufgrund der gespannten Spanneinheit zum ersten Plateau hin beschleunigt, wodurch der Kolben zum offenen Abgabeende hin bewegt wird.

Die erfindungsgemäße Vorrichtung ist bevorzugt als Selbstfüllerspritze ausgebildet. Unter einer Selbstfüllerspritze wird hier insbesondere eine Spritze verstanden, bei der während oder durch die Bewegung des Kolbens weg vom Abgabeende der Spritze das zu applizierende Fluid in den Zylinder gebracht wird und bei der durch die Bewegung des Zylinders zum Abgabeende hin das Fluid zum Applizieren abgegeben wird. Die erfindungsgemäße Vorrichtung ist bevorzugt als Selbstfüllerspritze zum nadellosen Applizieren (insbesondere intradermal, subkutan oder intramuskulär) bei Tieren und/oder Menschen ausgebildet.

Natürlich kann die erfindungsgemäße Vorrichtung auch so ausgebildet sein, dass sie als Selbstfüllerspritze mit einer Kanüle am Abgabeende ausgebildet ist.

Das erste Ventil kann als passives Ventil oder als aktives Ventil ausgebildet sein. Ferner kann das zweite Ventil als passives Ventil oder aktives Ventil ausgebildet sein. Unter einem passiven Ventil wird hier insbesondere verstanden, dass ein Öffnen und Schließen des Ventils durch einem im Zylinder erzeugten Über- oder Unterdruck aufgrund der Bewegung des Kolbens im Zylinder bewirkt wird. Unter einem aktiven Ventil wird hier insbesondere ein Ventl verstanden, dass mittels eines Stellglieds geöffnet und geschlossen werden kann. Unter einem geöffneten ersten bzw. zweiten Ventil wird hier insbesondere verstanden, dass die gewünschte Fluidverbindung zwischen Zylinder und Abgabeende bzw. Zuführkanal vorhanden ist. Unter einem geschlossenen ersten bzw. zweiten Ventil wird hier insbesondere verstanden, dass keine Fluidverbindung zwischen Zylinder und Abgabeende bzw. Zuführkanal vorhanden ist (oder die bei geöffnetem Ventil gewünschte Fluidverbindung unterbrochen oder gesperrt ist). Das erste Ventil kann als Rückschlagventil ausgebildet sein. Ferner kann das zweite Ventil als Rückschlagventil ausgebildet sein.

Die Vorrichtung kann so ausgebildet sein, dass eine Bewegung des Kolbens entlang der ersten Richtung einen Unterdruck im Zylinder erzeugt, so dass bei geöffnetem zweiten Ventil das zu applizierende Fluid über den Zuführkanal in den Zylinder gesaugt wird. Insbesondere kann der erzeugte Unterdruck das zweite Ventil öffnen. Ferner kann der durch die Bewegung des Kolbens entgegen der ersten Richtung zum offenen Abgabeende hin erzeugte Überdruck im Zylinder das erste Ventil öffnen, wodurch das zu applizierende Fluid im Zylinder über das offene Abgabeende zum Applizieren abgegeben wird.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung anhand von Ausführungsbeispielen unter Bezugnahme auf die beigefügten Zeichnungen, die ebenfalls erfindungswesentliche Merkmale offenbaren, noch näher erläutert. Diese Ausführungsbeispiele dienen lediglich der Veranschaulichung und sind nicht als einschränkend auszulegen. Beispielsweise ist eine Beschreibung eines Ausführungsbeispiels mit einer Vielzahl von Elementen oder Komponenten nicht dahingehend auszulegen, dass alle diese Elemente oder Komponenten zur Implementierung notwendig sind. Vielmehr können andere Ausführungsbeispiele auch alternative Elemente und Komponenten, weniger Elemente oder Komponenten oder zusätzliche Elemente oder Komponenten enthalten. Elemente oder Komponenten verschiedener Ausführungsbeispiele können miteinander kombiniert werden, sofern nichts anderes angegeben ist. Modifikationen und Abwandlungen, welche für eines der Ausführungsbeispiele beschrieben werden, können auch auf andere Ausführungsbeispiele anwendbar sein. Zur Vermeidung von Wiederholungen werden gleiche oder einander entsprechende Elemente in verschiedenen Figuren mit gleichen Bezugszeichen bezeichnet und nicht mehrmals erläutert. Von den Figuren zeigen:
- Fig. 1: eine perspektivische Ansicht eines Ausführungsbeispiels der erfindungsgemäßen Applizierungsvorrichtung 1;
- Fig. 2: eine Vorderansicht der Zylinder-Kolben-Anordnung 10 der Applizierungsvorrichtung 1;
- Fig. 3: eine Schnittansicht der Zylinder-Kolben-Anordnung 10 gemäß der Schnittlinie A-A in Fig. 2;
- Fig. 4: eine Schnittansicht der Zylinder-Kolben-Anordnung 10 entlang der Schnittlinie B-B in Fig. 3;
- Fig. 5: eine Schnittansicht der Zylinder-Kolben-Anordnung 10 gemäß der Schnittlinie C-C in Fig. 4;
- Fig. 6: eine isometrische Ansicht der Zylinder-Kolben-Anordnung 10, wobei die Vorrichtung gespannt ist und der Kolben in seiner hinteren Endposition steht;
- Fig. 7: eine isometrische Ansicht der Zylinder-Kolben-Anordnung 10, wobei der Kolben in seiner vorderen Endposition steht;
- Fig. 8: ein Diagramm zur Darstellung des Verlaufs der Rampenbahn 41, wobei entlang der x-Achse der Drehwinkel α und entlang der y-Achse der Hub entlang der Längsachse der Kolbenstange 25 aufgetragen ist;

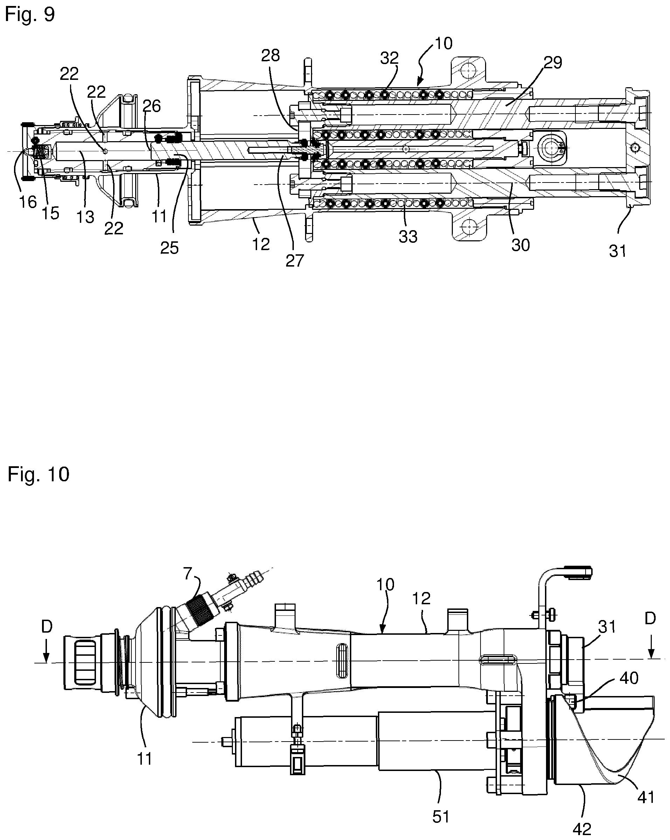

- Fig. 9: eine Schnittansicht der Kolben-Zylinder-Anordnung 10 im gespannten Zustand gemäß Fig. 6;
- Fig. 10: eine Seitenansicht der Kolben-Zylinder-Anordnung 10, bei der der Kolben in seiner vorderen Endposition steht;
- Fig. 11: eine Schnittansicht der Kolben-Zylinder-Anordnung 10 gemäß der Schnittlinie D-D in Fig. 10;
- Fig. 12: eine vergrößerte Schnittdarstellung des Vorderteils 11;
- Fig. 13: eine vergrößerte Detailansicht des Details E gemäß Fig. 12, wobei der Kolben 25 näher am offenen Abgabeende 14 positioniert ist im Vergleich mit der Darstellung Fig. 12;
- Fig. 14: eine vergrößerte Darstellung des Ringquerschnitts einer Ausführungsform des ersten Dichtelements 61, und
- Fig. 15-18: vergrößerte Darstellungen des Ringquerschnitts weiterer Ausführungsformen des ersten Dichtelementes 61.

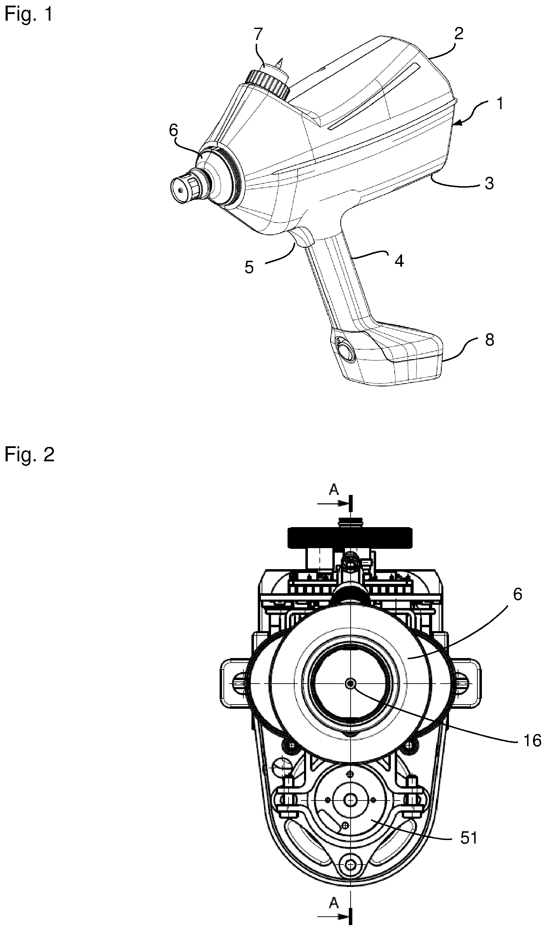

Bei dem in Fig. 1 gezeigten Ausführungsbeispiel umfasst die erfindungsgemäße Vorrichtung 1 zum Applizieren eines Fluids (z.B. einer Flüssigkeit) ein Gehäuse 2 mit einem Hauptabschnitt 3 und einem Griffabschnitt 4. Der Griffabschnitt 4 ist so ausgebildet, dass ein Benutzer die Vorrichtung 1 durch Umgreifen des Griffabschnittes 4 halten kann. Des Weiteren weist der Griffabschnitt 4 einen Auslöser 5 zum Betätigen der Vorrichtung 1 auf. Am vorderen Ende des Hauptabschnittes 3 ist ein Abgabebereich 6 ausgebildet. Des Weiteren weist die Vorrichtung 1 am oberen Bereich des Hauptabschnittes 3 einen Anschluss 7 auf, mit dem z.B. ein Schlauch oder ein Behälter verbunden werden kann. Über den Schlauch kann das zu applizierende Fluid zugeführt wird. In gleicher Weise kann im Behälter das zu applizierende Fluid enthalten sein.

Der Griffabschnitt 4 geht an seinem vom Hauptabschnitt 3 wegweisenden Ende in einen Fuß 8 über, in dem z.B. eine Stromversorgung (beispielsweise ein Akku) für die Vorrichtung 1 enthalten sein kann.

Die erfindungsgemäße Vorrichtung 1, die auch als Applizierungsvorrichtung 1 bezeichnet werden kann, ist bei dem hier beschriebenen Ausführungsbeispiel zum nadellosen Applizieren des Fluids bei einem Tier ausgebildet. Bei dem Applizieren handelt es sich bevorzugt um ein intramuskuläres Injizieren des Fluids, das z.B. ein Arzneimittel, ein Impfstoff oder dergleichen sein kann.

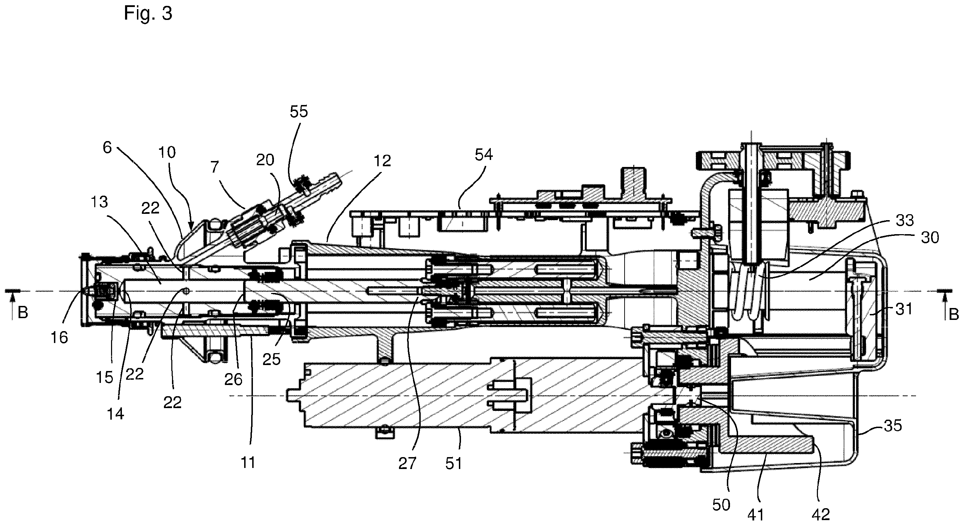

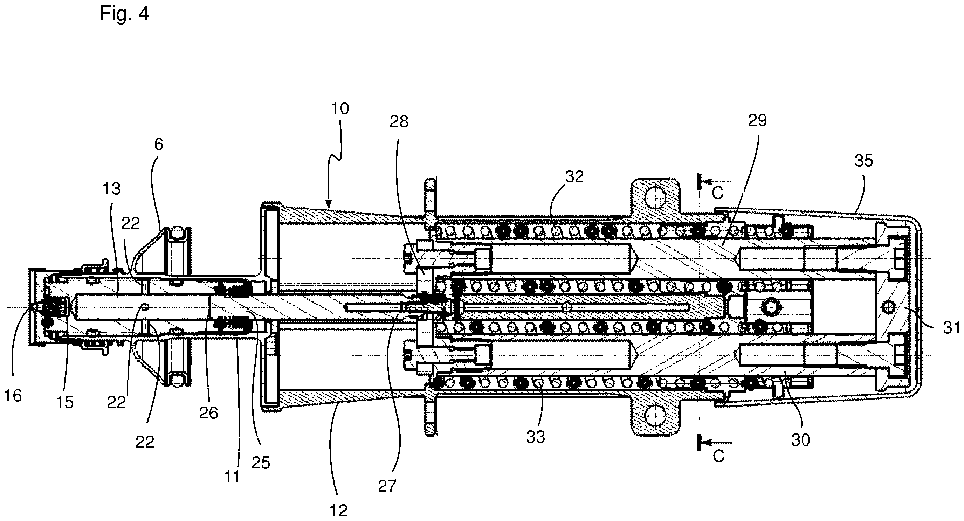

Die Applizierungsvorrichtung 1 weist eine nachfolgend noch im Detail beschriebene Zylinder-Kolben-Anordnung 10 (Fig. 3 und 4) auf und ist als Selbstfüllertyp derart ausgebildet, dass durch eine Kolbenbewegung zum Abgabebereich 6 hin ein Ausspritzen des Fluids bewirkt wird und durch eine entgegengesetzte Bewegung des Kolbens ein Auffüllen des Zylinders mit dem Fluid für den nächsten Ausspritzvorgang bewirkt wird.

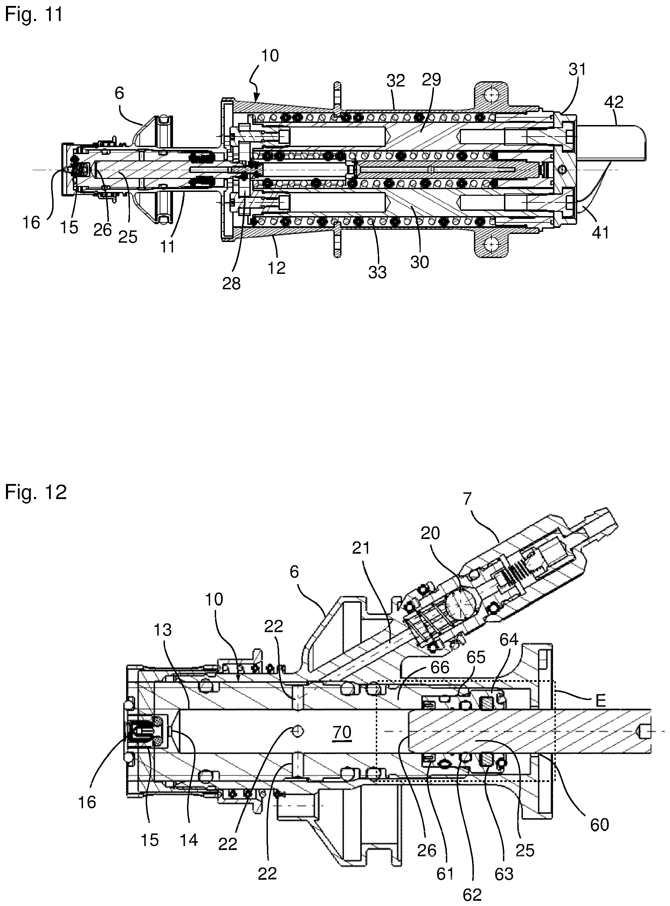

In Fig. 2 bis 5 ist die gesamte Zylinder-Kolben-Anordnung 10 ohne Gehäuse 2 dargestellt. Die Zylinder-Kolben-Anordnung 10 umfasst ein Vorderteil 11 und ein damit verbundenes Hinterteil 12. Das Vorderteil 11 umfasst einen Zylinder 13 zur Aufnahme des Fluids, der ein offenes Abgabeende 14 aufweist, in dem ein erstes Rückschlagventil 15 sitzt, das in Fluidverbindung mit einer Düse 16 steht. Das erste Rückschlagventil 15 ist auch gut in der vergrößerten Schnittdarstellung des Vorderteils 11 in Fig. 12 zu erkennen und ist so ausgebildet, dass eine Abgabe des Fluids aus dem Zylinder 13 über das erste Rückschlagventil 15 und die Düse 16 ermöglicht wird. Ein Ansaugen von Luft oder Flüssigkeit über die Düse 16 und über das erste Rückschlagventil 15 ist nicht möglich. In dieser Richtung schließt das Rückschlagventil 15.

An dem Vorderteil 11 ist ferner der Anschluss 7 ausgebildet, in dem ein zweites Rückschlagventil 20 (Fig. 12) sitzt, das eine Fluidverbindung vom Anschluss 7 zum Zylinder 13 ermöglicht und eine Fluidverbindung in entgegengesetzter Richtung sperrt. Der Anschluss 7 weist einen Kanal 21 (bzw. Zuführkanal 21) auf, der über mehrere radiale Bohrungen 22 in den Zylinder 13 mündet.

Das zweite Rückschlagventil 20 kann als Einlassventil und das erste Rückschlagventil 15 kann als Auslassventil bezeichnet werden.

Im Zylinder 13 ist ein Kolben 25 mit einem zum offenen Abgabeende 14 hin weisenden Kolbenende 26 geführt, wobei der Kolben 25 in den Schnittdarstellungen von Fig. 3, 4 und 12 in seiner hinteren Endposition steht. In dieser Position ist der Zylinder 13 mit dem abzugebenden Fluid gefüllt. Der Kolben 25 ist hier als Stange mit konstantem Querschnitt ausgebildet, wobei der Teil der Stange, der im Zylinder 13 hin und her bewegt ist, als Kolben 25 bezeichnet wird, und der Teil der Stange, der aus dem Zylinder 13 nach hinten raussteht, als Kolbenstange 27 bezeichnet wird.

Das von dem offenen Abgabeende 14 wegweisende hintere Ende (gut in Fig. 4 zu erkennen) der Kolbenstange 27 ist über eine Platte 28 mit einer ersten Führungsstange 29 und einer zweiten Führungsstange 30 verbunden, die sich parallel zueinander und parallel zum Kolben 25 erstrecken und die im Hinterteil 12 geführt sind. Die von der Platte 28 wegweisenden Enden der Führungsstangen 29 und 30 sind mit einem Mitnehmer 31 verbunden.

Ferner ist für jede Führungsstange 29 und 30 eine Druckfeder 32, 33 (z.B. Schraubenfeder) angeordnet, deren vordere Enden jeweils an der Platte 28 und deren hintere Enden jeweils an einem Anschlag des Hinterteils 12 anliegen. In der in Fig. 3 und 4 gezeigten Stellung des Kolbens 26 sind die Federn 32, 33 gespannt.

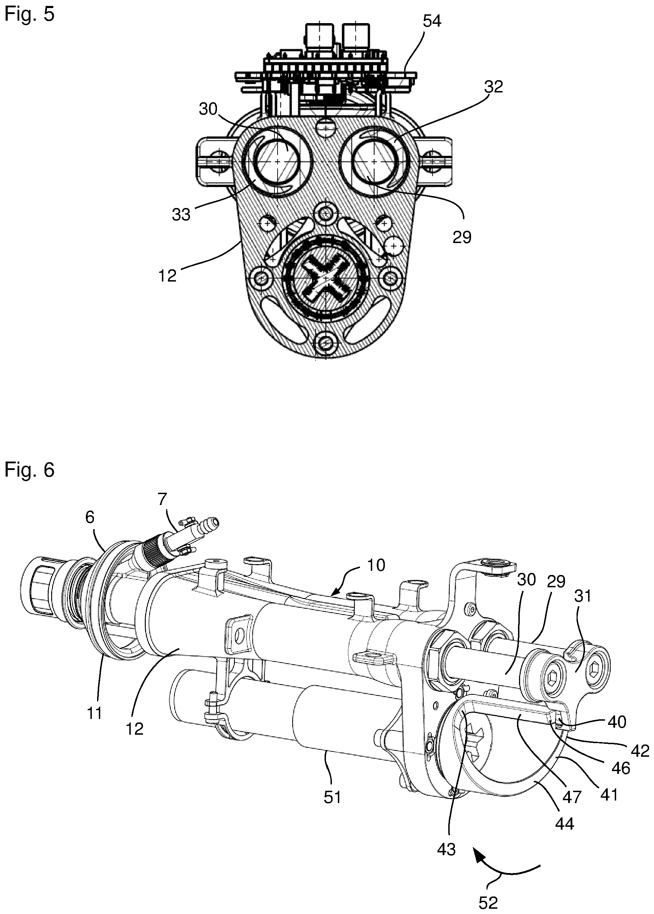

Am hinteren Ende des Hinterteils 12 ist eine Abdeckung 35 vorgesehen, die in der isometrischen Ansicht der Zylinder-Kolben-Anordnung 10 gemäß Fig. 6 nicht dargestellt ist, so dass der Mitnehmer 31 gut erkennbar ist. Der Mitnehmer 31 weist eine drehbar gelagerte Walze 40 auf, wobei sich die Drehachse der Walze 40 im Wesentlichen senkrecht zur Längsachse des stangenförmigen Kolbens 25 erstreckt.

Die Walze 40 läuft auf einer Rampenbahn 41 einer sich unter der Walze 40 hindurch drehenden Rampe 42, wobei die Rampenbahn 41 eine einzige Windung aufweist, wie insbesondere Fig. 6 bis 8 zu entnehmen ist.

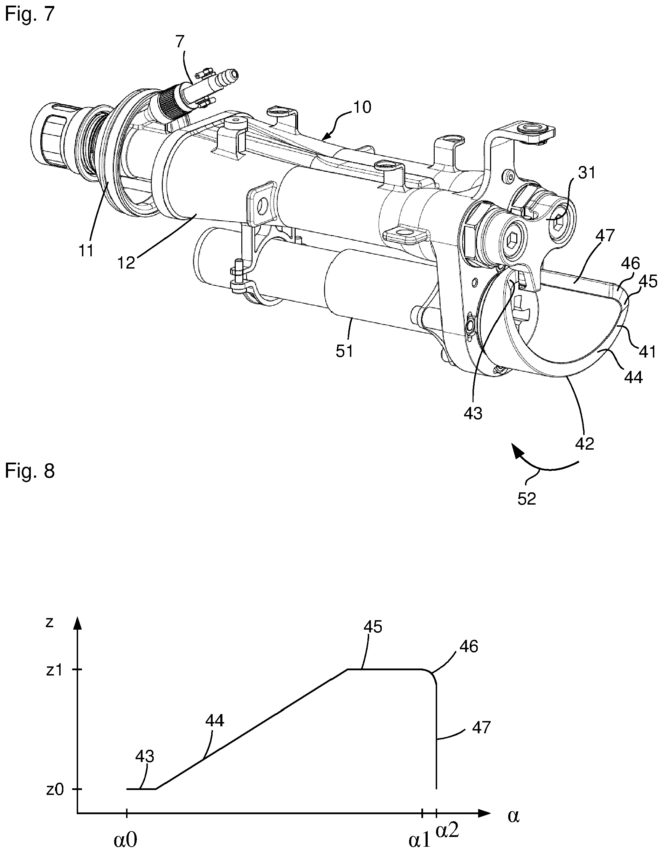

In Fig. 8 ist der Drehwinkel α gegenüber dem Gangunterschied z parallel zur Längsrichtung des Kolbens 25 aufgetragen, wobei davon ausgegangen wird, dass bei einem Drehwinkel von a0 = 0° die geringste Ganghöhe z0 vorliegt und der Kolben 26 somit in einer vorderen Endposition steht, in der der Abstand des vorderen Kolbenendes 26 zum offenen Abgabeende 14 minimal ist. Diese Stellung des Kolbens 25 ist beispielsweise in der Schnittdarstellung gemäß Fig. 11 gezeigt.

Die Rampenbahn 41 weist ein unteres Plateau 43 auf, an dem sich ein Steigungsbereich 44 anschließt, der bis zum oberen Plateau 45 verläuft. An das obere Plateau 45 schließt sich ein Übergangsbereich 46 an, der in eine Sprungflanke 47 (Drehwinkel a1) mündet, die wieder zum ersten Plateau 43 führt. Somit entspricht der Drehwinkelbereich von a0 bis a2 gleich 360°.

Die Sprungflanke 47 zeichnet sich dadurch aus, dass sie quasi senkrecht verläuft, da sie sich beim einem Drehwinkel (hier a2) von der Höhe z1 zur Höhe z0 erstreckt. Der Übergangsbereich 46 ist somit der Drehwinkelbereich, bei dem die Höhe z1 ausgehend vom oberen Plateau 45 kontinuierlich abnimmt, bis der Drehwinkel a2 (= Sprungflanke 47) erreicht ist. Somit deckt der Drehwinkelbereich von a1 zu a2 den Übergangsbereich 46 ab.

Die Rampe 42 ist über eine Kupplung 50 mit einem Motor 51 verbunden (Fig. 3), der die Rampe 42 in einer ersten Drehrichtung 52 dreht (Figuren 6 und 7). Wenn nun der Motor 51 die Rampe 42 ausgehend von der in Fig. 6 gezeigten Position, in der die Zylinder-Kolben-Anordnung 10 gespannt ist, weiter in der ersten Drehrichtung 52 dreht (da ein Benutzer den Auslöser 5 betätigt hat), läuft die Walze 40 über den Übergangsbereich 46 und fällt dann entlang der Sprungflanke 47 in Richtung zum unteren Plateau 43, da die gespannten Druckfedern 32 und 33 die Platte 28 in Richtung hin zum offenen Abgabeende 14 beschleunigen, wodurch der mit der Platte 28 verbundene Kolben 25 ebenfalls zum vorderen Abgabeende 14 hin bewegt wird und dabei das im Zylinder 13 enthaltene Fluid über das erste Rückschlagventil 15 und die Düse 16 zur intramuskulären Injektion bei einem Tier ausgespritzt wird. Die Applizierungsvorrichtung 1 ist dabei so ausgelegt, dass das Fluid sicher die Haut durchdringt und in den darunter liegenden Muskel appliziert wird. Das vordere Kolbenende 26 steht dann in seiner vorderen Endposition, wie z.B. in der Schnittdarstellung in Fig. 11 gezeigt ist. Die Applizierungsvorrichtung 1 ist bevorzugt so ausgelegt, dass in der vorderen Endposition des vorderen Kolbenendes 26 der Mitnehmer 31 am hinteren Ende des Hinterteils 12 anliegt, wodurch das hintere Ende des Hinterteils 12 einen Anschlag für den Mitnehmer 31 bildet. In dieser Position liegt noch ein gewünschter Mindestabstand zwischen der Walze 40 und der Rampenbahn 41 vor, so dass von der Walze 40 das untere Plateau 43 der Rampenbahn 41 nicht erreicht wird. Damit kann verhindert werden, dass die Walze 40 am Ende des Ausspritzvorgangs auf die Rampenbahn 41 trifft, was zu einer Beschädigung der Walze 40 führen könnte.

Nach dem Ausspritzvorgang wird mittels des Motors 51 die Rampe 42 wieder in der ersten Drehrichtung 52 gedreht, so dass sobald die Walze 40 im Steigungsbereich 44 Kontakt mit der Rampenbahn 41 erlangt ein weiteres Drehen dazu führt, dass der Mitnehmer 31 entlang der Längsrichtung des Kolbens 25 vom offenen Abgabeende 14 weg bewegt wird, wodurch die Druckfedern 32, 33 wieder gespannt werden und ihre maximale Spannung erreichen, wenn die Walze 40 das obere Plateau 45 erreicht. Diese Bewegung des Mitnehmers 31 führt aufgrund der mechanischen Verbindung des Mitnehmers 31 mit den Führungsstangen 29 und 30, der Platte 28 und der Kolbenstange 25 dazu, dass auch der Kolben 25 und somit das vordere Kolbenende 26 in einer Richtung weg vom offenen Abgabeende im Zylinder 13 bewegt und somit ein Unterdruck aufgebaut wird. Sobald der aufgebaute Unterdruck so groß ist, dass das Einlassventil 20 öffnet, wird das Fluid durch das Einlassventil 20 und die radialen Bohrungen 22 in den Zylinder 13 gesaugt, so dass der Zylinder 13 mit dem Fluid befüllt wird.

Wenn die Walze 40 (die auch als Nocken oder Rolle bezeichnet werden kann) das obere Plateau 45 erreicht hat, stoppt der Motor 51, so dass die Zylinder-Kolben-Anordnung 10 gespannt und damit die Applizierungsvorrichtung 1 bereit zum nächsten Applizierungsvorgang ist, der durch Betätigen des Auslösers 5 durchgeführt werden kann.

Die Platte 28, die Federn 32, 33 samt Führungsstangen 29, 30, der Mitnehmer 31 mit Walze 40, die Rampe 42 bilden zusammen mit Motor 51 und Kupplung 50 eine Spannvorrichtung S zum Spannen der Zylinder-Kolben-Anordnung 10.

Ferner umfasst die Applizierungsvorrichtung 1 eine Steuereinheit 54 zum Ansteuern des Motors 51 sowie allen weiteren elektrischen Komponente der Vorrichtung 1. In Fig. 3 ist eine Platine mit der Steuereinheit 54 gezeigt.

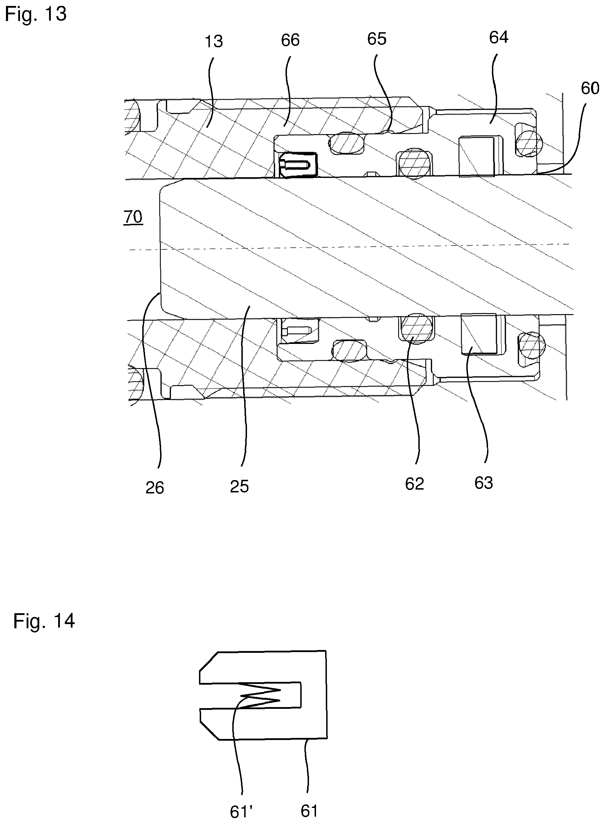

Bei der Applizierungsvorrichtung 1 ist es wichtig, dass das hintere Ende 60 des Zylinders 13 (Figuren 12 und 13) gut abgedichtet ist, da bei der bestimmungsgemäßen Verwendung der Applizierungsvorrichtung 1 hohe, stark schwellende Drücke sowie Unterdruck auftreten. Im Zylinder 13, der einen Druckraum 70 aufweist, herrscht sowohl Überdruck (beim Applizieren des Fluids) als auch Unterdruck (beim Ansaugen von neuem Fluid für den nächsten Applizierungsvorgang). Dabei ist die Geschwindigkeit des Kolbens 25 beim Ansaugen viel langsamer als beim Applizieren. Normale O-Ring-Dichtungen sind in der Regel nicht für hohe dynamische, stark schwellende Drücke ausgelegt, da solche O-Ringe dabei schnell verschleißen. Darüber hinaus kann Abrieb der O-Ring-Dichtungen in nachteiliger Weise beispielsweise das Auslassventil 16 verstopfen. Dies kann zu einer Fehlfunktion und einer daher notwendigen Reparatur sowie zu einem unerwünscht höheren Wartungsaufwand führen.

Erfindungsgemäß sind daher ein erstes Dichtelement 61 und ein davon entlang der ersten Richtung (in Fig. 12 von links nach rechts) beabstandetes zweites Dichtelement 62 angeordnet. Das erste Dichtelement 61 ist als einfachwirkendes Dichtelement ausgebildet, das das hintere Ende 60 des Zylinders 13 gegenüber Überdruck im Zylinder 13 abdichtet. Das erste Dichtelement 61 kann ringförmig sein und einen U-förmigen Querschnitt (oder V-förmigen Querschnitt) aufweisen, wobei das offene Ende des U-förmigen Querschnitts (oder V-förmigen Querschnitts) zum vorderen Ende 14 bzw. zum offenen Abgabeende 14 hin offen ist. Bei Überdruck im Druckraum 61 werden dann die beiden Schenkel des U-förmigen Querschnitts (oder V-förmigen Querschnitts) des ersten Dichtelements 61 radial auseinander gedrückt, so dass die gewünschte Dichtwirkung sichergestellt werden kann. Das erste Dichtelement 61 kann im U-förmigen Querschnitt (oder V-förmigen Querschnitt) ein Federelement 61' aufweisen, das die beiden Schenkel des U-förmigen Querschnitts (oder V-förmigen Querschnitts) bereits in radialer Richtung auseinander drückt, um die gewünschte Dichtwirkung zu verstärken, wie in Fig. 14 schematisch dargestellt ist.

Da das einfachwirkende Dichtelement 61 jedoch das hintere Ende 60 des Zylinders 13 nicht oder nur schlecht gegen Unterdruck in der Druckkammer 70 abdichtet, ist das zweite Dichtelement 62 vorgesehen. Im hier beschriebenen Ausführungsbeispiel ist das zweite Dichtelement 62 als doppelwirkendes Dichtelement 62 ausgebildet (es kann beispielsweise eine O-Ring-Dichtung sein), die das hintere Ende 60 des Zylinders 13 gegen Unterdruck im Druckraum 70 abdichtet.

Da das erste Dichtelement 61 das hintere Ende 60 bei Überdruck im Druckraum 70 abdichtet, liegen im Vergleich zu bisher bekannten Lösungen am zweiten Dichtelement 62 im Fall des Überdrucks in der Druckkammer 70 nicht mehr so große Drücke an, so dass der unerwünschte Verschleiß einer solchen O-Ring-Dichtung 62 stark verringert ist.

Ferner kann noch ein zwischen dem Zylinder 13 und dem Kolben 25 angeordneter Schmierring 63 vorgesehen sein. Dabei kann es sich beispielsweise um einen Filzring oder einen schwammförmiges Material handeln. Der Schmierring 63 kann beispielsweise mit Öl oder Fett beaufschlagt sein, um eine möglichst gute Schmierung des Kolbens 25 zu gewährleisten.

Bei der in Figuren 12 und 13 gezeigten Ausführungsform sind der erste und zweite Dichtring 61 und 62 sowie der optional vorgesehene Schmierring 63 ortsfest am Zylinder 13 angeordnet, so dass sich der Kolben 25 relativ zum ersten und zweiten Dichtring 61 und 62 sowie zum optional vorgesehenen Schmierring 63 bewegt. Es ist natürlich auch möglich, den ersten Dichtring 61 und/oder den zweiten Dichtring 62 (bevorzugt dann beide Dichtringe 61 und 62) ortsfest am Kolben 25 anzuordnen, so dass sich der Kolben 25 zusammen mit den Dichtringen 61 und 62 bewegt. Auch der Schmierring 63 kann am Kolben 25 ortsfest angeordnet sein.

Wie in der vergrößerten Detaildarstellung in Figur 13 gut erkennbar ist, sind der erste und zweite Dichtring 61 und 62 sowie der optionale Schmierring 63 in einem hinteren Zylinderteil 64, dessen hinteres Ende auch das hintere Ende 60 des Zylinders 13 bildet, angeordnet, der über einen Schnappverschluss 65 mit dem vorderen Zylinderteil 66 des Zylinders 13 verbunden ist.

Damit hat man einen kompakten Zylinder 13, der als Ganzes aus der Vorrichtung entfernbar ist. Diesen kompakten Zylinder 13 kann man dann in seine Teile trennen und beispielsweise nur den hinteren Zylinderteil 64 samt den Dichtringen 61 und 62 und dem Schmierring 63 austauschen. Natürlich ist es auch möglich, lediglich den ersten und zweiten Dichtring 61 und 62 und den optionalen Schmierring 63 bei einer Wartung auszuwechseln und den hinteren Zylinderteil 64 erneut zu verwenden.

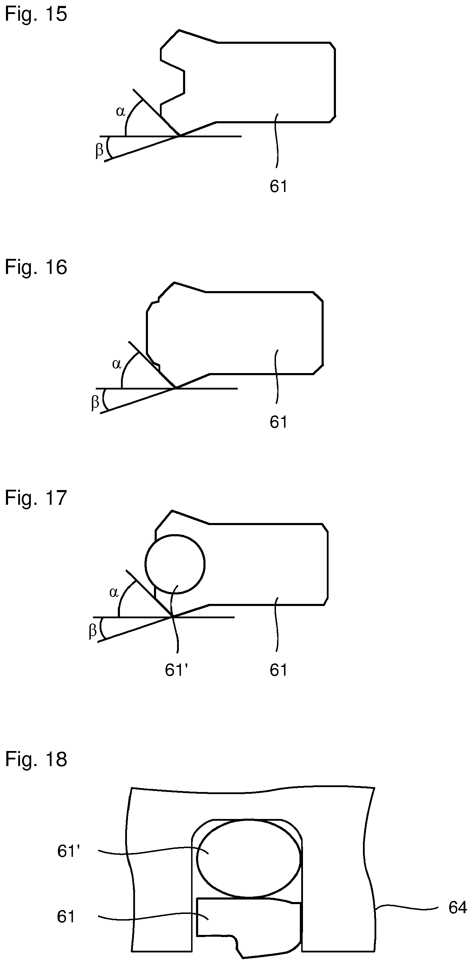

Bei der in Fig. 15 gezeigten Ausführungsform ist der erste Dichtring 61 als Nutring ausgebildet. Dabei kann insbesondere die stumpfwinklige Dichtkante mit der Nutringoberfläche einen größeren Winkel α aufweisen als die Außenseite der Dichtkante mit dem flacheren Winkel β. Als Material kann hartes Polyurethan verwendet werden.

In Fig. 16 ist einen Abwandlung des Nutrings von Fig. 15 gezeigt.

In Fig. 17 ist der Nutring von Fig. 15 so abgewandelt, dass er zweiteilig ausgebildet ist und zusätzlich den elastischen O-Ring 61' aufweist, der die Dichtkanten radial nach außen drückt und für eine gewünschte Vorspannung sorgt.

In Fig. 18 ist eine weitere mögliche Ausführungsform des ersten Dichtelementes 61 gezeigt. Bei dieser Ausführungsform ist ein Dichtring 61 (z.B. aus PTFE oder PTFE-Compound) vorgesehen, der an seiner Außenseite einen Elastomer-O-Ring 61' aufweist. Der Elastomer-O-Ring 61' dient zu einem Andrücken des Dichtrings 61 an den Kolben 25. Dazu sitzt das derart ausgebildete erste Dichtelement 61 (mit dem Elastomer-O-Ring 61') in einer entsprechenden Aufnahmenut im hinteren Zylinderteil 64.

## Patentansprüche

1. Vorrichtung zum Applizieren eines Fluids, mit
einem Zylinder (13), der ein offenes Abgabeende (14) aufweist,
einem im Zylinder (13) zwischen einer vorderen und hinteren Endposition verschiebbaren Kolben (25), der mit einer Kolbenstange (27) verbunden ist, die entlang einer ersten Richtung über ein dem offenen Abgabeende (14) entgegengesetztes hinteres Ende (60) des Zylinders (13) heraussteht,
einem das offene Abgabeende (14) verschließenden ersten Ventil (15),
einem mit dem Zylinder (13) verbundenen Zuführkanal (21),
und einem den Zuführkanal (21) verschließenden zweiten Ventil (20),
wobei bei geöffnetem zweiten Ventil (20) zu applizierendes Fluid über den Zuführkanal (21) in den Zylinder (13) gebracht werden kann, und
wobei eine Bewegung des Kolbens (26) entgegen der ersten Richtung zum offenen Abgabeende (14) hin einen Überdruck im Zylinder (13) erzeugt, so dass bei geöffnetem ersten Ventil (15) das zu applizierende Fluid im Zylinder (13) über das offene Abgabeende (14) zum Applizieren abgegeben wird,
wobei zur Abdichtung des hinteren Endes (60) des Zylinders (13) zwischen dem Kolben (26) und dem Zylinder (13) ein erstes Dichtelement (61) und ein davon entlang der ersten Richtung beabstandetes zweites Dichtelement (62) angeordnet sind,
wobei das erste Dichtelement (61) als einfachwirkendes Dichtelement ausgebildet ist, das das hintere Ende (60) des Zylinders (13) gegenüber Überdruck im Zylinder (13) abdichtet, und
wobei das zweite Dichtelement (62) als einfachwirkendes oder doppelwirkendes Dichtelement ausgebildet ist, das das hintere Ende (60) des Zylinders (13) mindestens gegenüber Unterdruck im Zylinder (13) abdichtet.

2. Vorrichtung nach Anspruch 1, bei der
das erste Dichtelement (61) ringförmig ausgebildet ist und einen U-förmigen oder V-förmigen Ringquerschnitt aufweist, wobei das offene Ende des U-förmigen oder V-förmigen Ringquerschnitts zum offenen Abgabeende (14) hinweist.

3. Vorrichtung nach Anspruch 2, bei der das erste Dichtelement (61) ein im U-förmigen oder V-förmigen Ringquerschnitt angeordnetes Federelement (61') aufweist, das die beiden Schenkel des U-förmigen oder V-förmigen Ringquerschnitts auseinanderdrückt.

4. Vorrichtung nach einem der obigen Ansprüche, bei der
das erste Dichtelement (61) einen ringförmigen Dichtteil (61) sowie ein Federelement (61') aufweist, das den ringförmigen Dichtteil (61) mit einer radial nach innen gerichteten Spannung beaufschlagt.

5. Vorrichtung nach Anspruch 4, bei der
der ringförmige Dichtteil (61) aus einem Thermoplast, PTFE oder einem PTFE Compound und das Federelement (61') aus einem Elastomer hergestellt ist.

6. Vorrichtung nach einem der obigen Ansprüche, bei der
das zweite Dichtelement (62) als O-Ring-Dichtung ausgebildet ist.

7. Vorrichtung nach einem der obigen Ansprüche, bei der
das erste Dichtelement (61) und das zweite Dichtelement (62) ortsfest am Zylinder (13) angeordnet sind, so dass sich der Kolben (25) relativ zum ersten und zweiten Dichtelement (61, 62) bewegt.

8. Vorrichtung nach einem der obigen Ansprüche, bei der
der Zylinder (13) einen vorderen Zylinderabschnitt (66) und einen damit lösbar verbundenen hinteren Zylinderabschnitt (64), dessen hinteres Ende das hintere Ende (60) des Zylinders (13) bildet, aufweist,
wobei das erste und zweite Dichtelement (61, 62) ortsfest am hinteren Zylinderabschnitt (64) angeordnet sind.

9. Vorrichtung nach Anspruch 8, bei der
der hintere Zylinderabschnitt (64) mit dem vorderen Zylinderabschnitt (66) lösbar verbunden ist.

10. Vorrichtung nach Anspruch 9, bei der
die lösbare Verbindung des hinteren Zylinderabschnitts (64) mit dem vorderen Zylinderabschnitt (66) mittels eines Schnappverschlusses realisiert ist.

11. Vorrichtung nach einem der obigen Ansprüche, bei der
ein Schmierring (63) vorgesehen ist, der vom zweiten Dichtelement (62) in der ersten Richtung beabstandet angeordnet ist.

12. Vorrichtung nach einem der obigen Ansprüche, bei der
das erste Ventil (15) als Rückschlagventil und/oder das zweite Ventil (20) als Rückschlagventil ausgebildet sind/ist.
